# EUROPÄISCHE PATENTSCHRIFT

(11) **EP 1 715 056 B1**
(45) Veröffentlichungstag und Bekanntmachung des Hinweises auf die Patenterteilung: **17.07.2013**
(21) Anmeldenummer: 06112245.3
(22) Anmeldetag: 05.04.2006
(51) Int. Cl.: C12P 13/04

(54) **Verfahren zur Herstellung von L-Aminosäuren unter Verwendung verbesserter Stämme der Familie Enterobacteriaceae**
Process for the production of L-amino acids using improved Enterobacteriaceae strains
Procédé de production d'acides aminés L utilisant des souches améliorées de la famille Enterobacteriaceae

(30) Priorität: 23.04.2005 DE 102005019040
(43) Veröffentlichungstag der Anmeldung: 25.10.2006
(73) Patentinhaber: Evonik Degussa GmbH, 45128 Essen (DE)
(72) Erfinder: Rieping, Dr., Mechthild, 33619 Bielefeld (DE)

(56) Entgegenhaltungen:
- WO-A-2005/090589
- BLATTNER F R ET AL: "THE COMPLETE GENOME SEQUENCE OF ESCHERICHIA COLI K-12" SCIENCE, AMERICAN ASSOCIATION FOR THE ADVANCEMENT OF SCIENCE,, US, Bd. 277, 5. September 1997 (1997-09-05), Seiten 1453-1462, XP002069950 ISSN: 0036-8075
- ZENG G ET AL: "Repressor for the sn-glycerol 3-phosphate regulon of Escherichia coli K-12: Primary structure and identification of the DNA-binding domain" JOURNAL OF BACTERIOLOGY, WASHINGTON, DC, US, Bd. 178, Nr. 24, Dezember 1996 (1996-12), Seiten 7080-7089, XP002341250 ISSN: 0021-9193
- KRAMER R: "Genetic and physiological approaches for the production of amino acids" JOURNAL OF BIOTECHNOLOGY, ELSEVIER SCIENCE PUBLISHERS, AMSTERDAM, NL, Bd. 45, Nr. 1, 12. Februar 1996 (1996-02-12), Seiten 1-21, XP004036833 ISSN: 0168-1656

## Beschreibung

Diese Erfindung betrifft ein Verfahren zur Herstellung von L-Aminosäuren, insbesondere L-Threonin und L-Tryptophan, wobei das Medium Glycerin als Kohlenstoffquelle enthält, unter Verwendung von rekombinanten Mikroorganismen der Familie Enterobacteriaceae, in denen das glpR-Gen abgeschwächt wird, und die eine verbesserte Fähigkeit zur Nutzung von Glycerin und damit zur Bildung und Anreicherung der L-Aminosäuren zeigen.

### Stand der Technik

L-Aminosäuren, insbesondere L-Threonin und L-Tryptophan, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet z.B. einen Stamm aus, der L-Tryptophan produziert.

Seit einigen Jahren werden ebenfalls Methoden der rekombinanten DNA-Technik zur Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden.

Die gesamte Fermentationsindustrie zur Herstellung von L-Aminosäuren läuft heute zumeist auf Glukose oder Saccharose als Kohlenstoffquelle, die bei der Verarbeitung von Agrarerzeugnissen erhalten werden. Da jedoch der Preis dieser Kohlenstoffquellen steigt, ist eine technologisch durchführbare Alternative für das Produzieren der L-Aminosäuren, bevorzugt L-Threonin und L-Tryptophan, eine verbesserte Nutzung eines preiswerteren Materials als alternativer Rohstoff zur Fermentation, wünschenswert.

Es ist bekannt, dass L-Aminosäure produzierende Mikroorganismen auf Glycerin als alleiniger Kohlenstoffquelle wachsen (US-A-5,175,107). Glycerin (Propantriol) ist ein natürlicher Bestandteil von Ölen und Fetten und verbindet als "Brücke" die Fettsäuremoleküle in den Triglyceriden. Das Glycerin-Molekül ist stark polar und daher gut wasserlöslich. Als Kuppelprodukt entsteht dieser wertvolle Rohstoff bei der Biodieselherstellung (Rapsmethylester) und wird in Kosmetika, pharmazeutischen Produkten, Lebensmitteln und für technische Anwendungen eingesetzt. Entscheidend für den Einsatz von Glycerin als Rohstoff zur Herstellung von Futtermittelkomponenten ist die Preiswürdigkeit. Es ist davon auszugehen, dass mit zunehmender Biodieselproduktion Glycerin für den Futtermittelbereich interessanter wird.

### Aufgabe der Erfindung

Die Erfinder haben sich die Aufgabe gestellt, neue Maßnahmen zur verbesserten fermentativen Herstellung von L-Aminosäuren, insbesondere L-Threonin und L-Tryptophan, bereitzustellen.

### Beschreibung der Erfindung

Gegenstand der Erfindung ist ein Verfahren zur fermentativen Herstellung von L-Aminosäuren, insbesondere L-Threonin und L-Tryptophan, wobei das Medium Glycerin als Kohlenstoffquelle enthält, unter Verwendung von rekombinanten Mikroorganismen der Familie Enterobacteriaceae, die insbesondere bereits L-Aminosäuren produzieren, und in denen mindestens das glpR-Gen oder für dessen Genprodukt kodierende Nukleotidsequenzen abgeschwächt, insbesondere ausgeschaltet wird bzw. werden, die eine verbesserte Fähigkeit zur Nutzung von Glycerin zeigen und die in verbesserter Weise L-Aminosäuren, insbesondere L-Threonin und L-Tryptophan, produzieren.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Threonin und L-Tryptophan.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfinderischen Maßnahmen durchgeführt werden.

Gegenstand der Erfindung ist ein Verfahren zur Herstellung von L-Aminosäuren durch Fermentation von rekombinanten Mikroorganismen der Familie Enterobacteriaceae, dadurch gekennzeichnet, dass man
a) die die gewünschte L-Aminosäure produzierenden Mikroorganismen, in denen mindestens das glpR-Gen oder für das entsprechende Genprodukt kodierende Nukleotidsequenzen oder Allele abgeschwächt, insbesondere ausgeschaltet wird oder werden, in einem Glycerin-haltigen Medium kultiviert unter Bedingungen, bei denen die gewünschte L-Aminosäure im Medium oder in den Zellen angereichert wird, und,
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Bei den insbesondere rekombinanten Mikroorganismen handelt es sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Als Elternstamm geeignete, L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4,278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Als Elternstamm geeignete, L-Threonin produzierende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als Elternstamm geeignete, L-Tryptophan produzierende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5, 756, 345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5, 939, 295)

L-Tryptophan produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabinoheptulosonat-7-Phosphat(DHAP)-Synthese, Verstärkung der Chorismat-Biosynthese.

Es wurde gefunden, dass Mikroorganismen der Familie Enterobacteriaceae nach Abschwächung, insbesondere Ausschaltung mindestens des glpR-Gens oder dessen Allelen, in verbesserter Weise L-Aminosäuren, insbesondere L-Threonin und L-Tryptophan produzieren.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden.

Aus den ebenfalls zur Familie Enterobacteriaceae gehörenden Salmonella typhimurium (Accession No.: NC 003197 (Sequenz des gesamten Genoms)), Shigella flexneri (Accession No.: NC 004337 (Sequenz des gesamten Genoms)) und Erwinia carotovora (Accession No.: NC 004547 (Sequenz des gesamten Genoms)) ist die Nukleotidsequenz für das glpR-Gen ebenso bekannt.

Es ist bekannt, dass durch wirtseigene Enzyme (Methionin-Aminipeptidase) die N-terminale Aminosäure Methionin abgespalten werden kann.

Die Gene und Aktivitäten des Glycerin-Stoffwechsels (glycerol metabolism) sind zusammenfassend auch bei Lin (In: Neidhardt (ed), Escherichia coli und Salmonella, American Society for Microbiology, Washington, D.C., USA: 307-342 (1996)) beschrieben.

Das glpR-Gen von Escherichia coli K12 wird unter anderem durch folgende Angaben beschrieben:
glpR-Gen:

| | |
|---|---|
| Bezeichnung: | Repressor des glp-Regulons |
| Referenz: | Blattner et al.; Science 277(5331): 1453-1474 (1997) |
| SEQ ID No.: | 1 |
| Accession No.: | U00096 (Region: 3557870-3558628) |
| Alternativer Genname: | b3423 |

Die Expression der Enzyme, die am Glycerintransport und - metabolismus beteiligt sind, wird strikt reguliert. Dies wird durch die strukturelle Organisation der glp-Gene ermöglicht. Das Glycerophosphatregulon (glp) besteht aus fünf Operons, die an drei verschiedenen Genortenauf dem E. coli-Chromosom liegen (Cozzarelli et al., Journal of Molecular Biology 31: 371-387 (1968)). Alle Gene des glp-Systems stehen unter der Kontrolle des GlpR-Repressors (Schweizer und Larson, Journal of Bacteriology 169:507-513 (1987)). Der Genlocus von glpR liegt bei 75 min und wird im Gegenuhrzeigersinn transkribiert (Lin, in: Neidhardt (ed), Escherichia coli und Salmonella, American Society for Microbiology, Washington, D.C., USA: 307-342 (1996)). Der Repressor bildet im aktiven Zustand ein Tetramer aus identischen Untereinheiten von 30 kDa (Larson et al., Journal of Biological Chemistry 262: 15869-15874 (1987)) und bindet spezifisch an die Kontrollregionen des glp-Regulons. Sekundärstrukturanalysen zeigen, dass die spezifische DNA-Bindung des Repressors durch ein Helix-Schleife-Helix-Motiv in der Nähe des N-Terminus möglich ist (Zeng et al., Journal of Bacteriology 178: 7080-7089 (1996)). Diese DNA-Bindung kann durch den Induktor G3P aufgelöst werden (Larson et al., Journal of Biological Chemistry 262: 15869-15874 (1987)). Durch Versuche mit einer Mutante mit einer inaktiven Glycerinkinase wurde gezeigt, dass für eine Induktion der glp-Gene die Phosphorylierung von Glycerin notwendig ist. Folglich ist G3P der alleinige Induktor (Hayashi und Lin, Journal of Molecular Biology 14: 515-521 (1965); Lin, Annu. Rev. Microbiology 30:535-578 (1976)). Als Anti-Induktor wurde D-Galaktose-1-Phosphat identifiziert (Sundarajan, Biochemistry 50:463-469 (1963)).

Ein Regulon ist eine Einheit von Genen, die zwar an verschiedenen Orten eines Genoms lokalisiert sind, deren Expression aber durch die gleichen Regulatorproteine gesteuert wird. Ein Operon ist eine Einheit von gemeinsam regulierten Genen an einem Genort.

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum glpR-Gen von Escherichia coli unter der SEQ ID No. 1 und die ebenfalls bekannten Sequenzen zum glpR-Gen von Salmonella typhimurium bzw. Shigella flexneri bzw. Erwinia carotovora unter der SEQ ID No. 3 bzw. SEQ ID No. 5 bzw. SEQ ID No. 7 dargestellt. Die von diesen Leserahmen kodierten Proteine sind als SEQ ID No. 2, SEQ ID No. 4, SEQ ID No. 6 und SEQ ID No. 8 dargestellt.

Die in den angegebenen Textstellen beschriebenen offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des glpR-Gens, welche funktionsneutrale Sinnmutationen enthalten, zählen unter anderem solche, die zu höchstens 30 oder zu höchstens 20 oder zu höchstens 10, bevorzugt zu höchstens 7 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 30, 20, 10, 7, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 15 Aminosäuren betreffen.

Zu den geeigneten Allelen gehören ferner solche die durch Hybridisierung, insbesondere unter stringenten Bedingungen unter Verwendung von SEQ ID No. 1, SEQ ID No. 3, SEQ ID No. 5 oder SEQ ID No. 7 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% oder mindestens 99,6% Identität zur Sequenz der eingesetzten Sonde bzw. zu den in SEQ ID No. 1, SEQ ID No. 3, SEQ ID No. 5 oder SEQ ID No. 7 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin mit Stämmen der Familie Enterobacteriaceae vorteilhaft sein, zusätzlich zur Abschwächung des glpR-Gens, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Weiterhin kann es für die Produktion von L-Aminosäuren, insbesondere L-Trytophan mit Stämmen der Familie Enterobacteriaceae vorteilhaft sein, zusätzlich zur Verstärkung eines oder mehrerer der Gene des Glycerin-Stoffwechsels (glycerol metabolism), ausgewählt aus der Gruppe glpA, glpB, glpC, glpD, glpE, glpF, glpG, glpK, glpQ, glpT, glpX, tpiA, gldA, dhaK, dhaL, dhaM, dhaR, fsa und talC, ein oder mehrere Enzyme des bekannten Tryptophan-Biosyntheseweges oder Enzyme der Serin-Biosynthese oder Enzyme für die Produktion von 3-deoxy-D-arabinoheptulosonat-7-Phosphat (DHAP) und Chorismat zu verstärken. Die Verwendung endogener Gene wird im Allgemeinen bevorzugt.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Rekombinante Mikroorganismen mit einer solchen Verstärkung werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfinderischen Maßnahmen durchgeführt werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin- und L-Tryptophan-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden.

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens eines oder mehrere der im folgenden genannten Gene oder für deren Genprodukte kodierende Nukleotidsequenzen oder Allele trägt.

So können beispielsweise für die Produktion von L-Aminosäuren, insbesondere L-Threonin, gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992)),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986)),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983)),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yibD von Escherichia coli (Accession Number AE000439 des National Center for Biotechnology Information (NCBI, Bethesda, MD, USA), DE102004005836.9),
   verstärkt, insbesondere überexprimiert werden.

Desweiteren können beispielsweise für die Produktion von L-Aminosäuren, insbesondere L-Tryptophan, gleichzeitig eines oder mehrere der Gene, ausgewählt aus der Gruppe
- mindestens ein Gen des für die Anthranilat-Synthase, die Antranilat-Phosphoribosyltransferase, die Phosphoribosylanthranilat-Isomerase, die Indol-3-Glycerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5, 168, 056),
- das für die Transketolase B kodierende tktB-Gen (US5, 168, 056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA), EP1449918),
   verstärkt, insbesondere überexprimiert werden.

Weiterhin kann es für die Produktion von L-Aminosäuren, insbesondere L-Threonin vorteilhaft sein, zusätzlich zur Abschwächung des glpR-Gens, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist, und
- das für die Aspartat Ammonium-Lyase kodierende aspA-Gen (WO 03/008603)
abzuschwächen, insbesondere auszuschalten oder die Expression zu verringern.

Weiterhin kann es für die Produktion von L-Aminosäuren, insbesondere L-Tryptophan vorteilhaft sein, zusätzlich zur Abschwächung des glpR-Gens, eines oder mehrere der Gene ausgewählt aus der Gruppe
- das für die Tryptophanase kodierende tnaA-Gen (US4,371,614),
- das für den Repressor des trp-Operons kodierende trpR-Gen (US4, 371, 614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),
- das für das Tryptophan spezifische Transportprotein kodierende mtr-Gen (US5, 756, 345),
- das für die Tryptophan-Permease kodierende tnaB-Gen (US5, 756, 345),
- das für den Transporter für aromatische Aminosäuren kodierende aroP-Gen (US5, 756, 345),
- das für die L-Serin Deaminase kodierende sdaA-Gen (EP0149539),
- das für die Glukose-6-Phosphat-Isomerase kodierende pgi-Gen (WO87/01130),
- das für die Tyrosin-Aminotransferase kodierende tyrB-Gen (WO87/01130)
abzuschwächen, insbesondere auszuschalten oder die Expression zu verringern.

Weiterhin kann es für die Produktion von L-Aminosäuren, insbesondere L-Threonin und L-Tryptophan vorteilhaft sein, zusätzlich zur Abschwächung des glpR-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden ist im Lehrbuch von Chemiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle wird Glycerin eingesetzt. Dieses kann einzeln oder als Mischung verwendet werden. Zugesetzt werden können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Ethanol und Methanol und organische Säuren wie z.B. Essigsäure, wobei der Anteil des Glycerins mindestens größer gleich (≥) 50% oder mindestens ≥ 75% oder mindestens ≥ 90%, mindestens ≥ 95%, bevorzugt 100% betragen soll.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung der L-Aminosäure in der Kulturbrühe. Die Kultur wird solange fortgesetzt, bis sich ein Maximum an L-Aminosäuren bzw. L-Threonin oder L-Tryptophan gebildet hat. Dieses Ziel wird normalerweise innerhalb von 10 Stunden bis 160 Stunden erreicht.

Aus der entnommenen Kulturbrühe können das L-Threonin oder das L-Tryptophan gewonnen, gesammelt oder konzentriert und gegebenenfalls gereinigt werden. Typische Methoden zur Reinigung der L-Aminosäuren sind die Ionenaustauschchromatographie und die Kristallisation. Hierdurch erhält man weitgehend reine L-Aminosäuren.

Es ist ebenfalls möglich aus der entnommenen Kulturbrühe (= Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% oder kleiner 3% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

## Patentansprüche

1. Verfahren zur Herstellung von L-Aminosäuren, **dadurch gekennzeichnet, dass** man folgende Schritte durchführt:
a) Fermentation der die gewünschte L-Aminosäure produzierenden Mikroorganismen der Familie Enterobacteriaceae, in denen man das glpR-Gen oder die dafür kodierende Nukleotidsequenz abschwächt, insbesondere ausschaltet, in einem Glycerin-haltigen Medium und
b) Anreicherung der gewünschten L-Aminosäure im Medium oder in den Zellen der Mikroorganismen.

2. Verfahren gemäß Anspruch 1, **dadurch gekennzeichnet, dass** man die gewünschte L-Aminosäure isoliert, wobei gegebenenfalls Bestandteile der Fermentationsbrühe und/oder die Biomasse in ihrer Gesamtheit oder Anteilen (> 0 bis 100 %) davon im Produkt verbleiben.

3. Verfahren gemäß den Ansprüchen 1 oder 2 zur Herstellung von L-Aminosäuren, insbesondere L-Threonin und L-Tryptophan, oder diese Verbindungen enthaltenden Futtermitteladditiven durch Fermentation von Mikroorganismen der Familie Enterobacteriaceae, **dadurch gekennzeichnet, dass** man in diesen das glpR-Gen oder dafür kodierende Nukleotidsequenzen abschwächt, insbesondere ausschaltet, einsetzt und das gewünschte Produkt isoliert.

4. Verfahren gemäß den Ansprüchen 1, 2, oder 3, **dadurch gekennzeichnet, dass** man Mikroorganismen einsetzt, in denen die Aktivität oder Konzentration des glpR Genprodukts auf 0 bis 75% der Aktivität oder Konzentration des Wildtyp-Proteins oder im Elternstamm senkt.

5. Verfahren gemäß den Ansprüchen 1, 2, oder 3, **dadurch gekennzeichnet, dass** man die Expression des Polynukleotids, das für das Produkt des glpR-Gens kodiert, abschwächt, insbesondere ausschaltet.

6. Verfahren gemäß den Ansprüchen 1, 2 oder 3, **dadurch gekennzeichnet, dass** man die regulatorischen und/oder katalytischen Eigenschaften des Polypeptids (Enzymproteins) verringert, für das das Polynukleotid des glpR-Gens kodiert.

7. Verfahren gemäß den Ansprüchen 1 bis 6, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
7.1 mindestens ein Gen des für die Aspartatkinase, die Homoserin-Dehydrogenase, die Homoserinkinase und die Threoninsynthase kodierenden thrABC-Operons,
7.2 das für die Pyruvat-Carboxylase kodierende pyc-Gen von Corynebacterium glutamicum,
7.3 das für die Phosphoenolpyruvat-Synthase kodierende pps-Gen,
7.4 das für die Phosphoenolpyruvat-Carboxylase kodierende ppc-Gen,
7.5 die für die Untereinheiten der Pryridin-Transhydrogenase kodierenden Gene pntA und pntB,
7.6 das für das Threoninresistenz vermittelnde Protein kodierende Gen rhtC,
7.7 das für das Threonin-Export-Carrier-Protein kodierende thrE-Gen aus Corynebacterium glutamicum,
7.8 das für die Glutamat-Dehydrogenase kodierende gdhA-Gen,
7.9 das für die Phosphohistidin-Protein-Hexose-Phosphotransferase kodierende ptsH-Gen,
7.10 das für das Enzym I des Phosphotransferase-Systems kodierende ptsI-Gen,
7.11 das für die Glucose-spezifische IIA Komponente kodierende crr-Gen,
7.12 das für die Glucose-spezifische IIBC Komponente kodierende ptsG-Gen,
7.13 das für die Cystein-Synthase A kodierende cysK-Gen,
7.14 das für den Regulator des cys-Regulons kodierende cysB-Gen,
7.15 das für das Flavoprotein der NADPH-Sulfit-Reduktase kodierende cysJ-Gen,
7.16 das für das Hämoprotein der NADPH-Sulfit-Reduktase kodierende cysI-Gen,
7.17 das für die Adenylylsulfat-Reduktase kodierende cysH-Gen,
7.18 das für die Decarboxylase Untereinheit der 2-Ketoglutarat Dehydrogenase kodierende sucA-Gen,
7.19 das für die Dihydrolipoyltranssuccinase E2 Untereinheit der 2-Ketoglutarat Dehydrogenase kodierende sucB-Gen,
7.20 das für die β-Untereinheit der Succinyl-CoA Synthetase kodierende sucC-Gen,
7.21 das für die α-Untereinheit der Succinyl-CoA Synthetase kodierende sucD-Gen, und
7.22 das Genprodukt des offenen Leserahmens (ORF) yibD von Escherichia coli
verstärkt, insbesondere überexprimiert.

8. Verfahren gemäss den Ansprüchen 1 bis 6, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
8.1 mindestens ein Gen des für die Anthranilat-Synthase, die Antranilat-Phosphoribosyltransferase, die Phosphoribosylanthranilat-Isomerase, die Indol-3-Glycerinphosphat-Synthase und die Tryptophan-Synthase kodierenden Tryptophan-Operons,
8.2 das für die 3-Phosphoglycerat-Dehydrogenase kodierende serA-Gen,
8.3 das für die Phosphoserinphosphatase kodierende serB-Gen,
8.4 das für die Phosphoserinaminotransferase kodierende serC-Gen,
8.5 das für die L-Tyrosin sensitive DHAP-Synthase kodierende aroF-Gen,
8.6 das für die L-Phenylalanin sensitive DHAP-Synthase kodierende aroG-Gen,
8.7 das für die L-Tryptophan sensitive DHAP-Synthase kodierende aroH-Gen,
8.8 das für die Phosphoenolpyruvat-Synthase kodierende pps-Gen,
8.9 das für die Phosphoenolpyruvat-Carboxykinase kodierende pckA-Gen,
8.10 das für die Transketolase A kodierende tktA-Gen,
8.11 das für die Transketolase B kodierende tktB-Gen, und
8.12 das Genprodukt des offenen Leserahmens (ORF) yddG von Escherichia coli
verstärkt, insbesondere überexprimiert.

9. Verfahren gemäss den Ansprüchen 1 bis 7, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
9.1 das für die Threonin-Dehydrogenase kodierende tdh-Gen,
9.2 das für die Malat-Dehydrogenase kodierende mdh-Gen,
9.3 das Genprodukt des offenen Leserahmens (ORF) yjfA von Escherichia coli,
9.4 das Genprodukt des offenen Leserahmens (ORF) ytfP von Escherichia coli,
9.5 das für die Phosphoenolpyruvat-Carboxykinase kodierende pckA-Gen,
9.6 das für die Pyruvat-Oxidase kodierende poxB-Gen,
9.7 das für den DgsA-Regulator des Phosphotransferase-Systems kodierende dgsA-Gen,
9.8 das für den Fructose-Repressor kodierende fruR-Gen,
9.9 das für den Sigma³⁸-Faktor kodierende rpoS-Gen, und
9.10 das für die Aspartat Ammonium-Lyase kodierende aspA-Gen
abschwächt, insbesondere ausschaltet oder die Expression verringert.

10. Verfahren gemäss den Ansprüchen 1 bis 6 oder 8, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
10.1 das für die Tryptophanase kodierende tnaA-Gen,
10.2 das für den Repressor des trp-Operons kodierende trpR-Gen,
10.3 das für die Chorismat-Mutase T und Prephenat-Dehydrogenase kodierende tyrA-Gen,
10.4 das für die Chorismat-Mutase P und Prephenat-Dehydrogenase kodierende pheA-Gen,
10.5 das für das Tryptophan spezifische Transportprotein kodierende mtr-Gen,
10.6 das für die Tryptophan-Permease kodierende tnaB-Gen,
10.7 das für den Transporter für aromatische Aminosäuren kodierende aroP-Gen,
10.8 das für die L-Serin Deaminase kodierende sdaA-Gen,
10.9 das für die Glukose-6-Phosphat-Isomerase kodierende pgi-Gen, und
10.10 das für die Tyrosin-Aminotransferase kodierende tyrB-Gen
abschwächt, insbesondere ausschaltet oder die Expression verringert.

11. Verfahren gemäss den Ansprüchen 1 bis 6, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Arginin und L-Homoserin herstellt.

12. Verfahren gemäss den Ansprüchen 1 bis 6, **dadurch gekennzeichnet, dass** man L-Aminosäuren, ausgewählt aus der Gruppe L-Isoleucin, L-Valin, L-Methionin, L-Homoserin und L-Lysin herstellt.

## Claims

1. Process for the production of L-amino acids, **characterized in that** the following steps are carried out:
a) fermentation in a glycerol-containing medium of the microorganisms producing the desired L-amino acid of the family Enterobacteriaceae in which the glpR gene or the nucleotide sequence encoding it is attenuated, in particular turned off, and
b) enrichment of the desired L-amino acid in the medium or in the cells of the microorganisms.

2. Method according to Claim 1, **characterized in that** the desired L-amino acid is isolated, wherein, optionally, components of the fermentation broth and/or the biomass remain in the product in totality, or fractions (> 0 to 100%) thereof.

3. Process according to Claims 1 or 2 for the production of L-amino acids, in particular L-threonine and L-tryptophan, or feed additives containing these compounds, by fermentation of microorganisms of the family Enterobacteriaceae, **characterized in that**, therein, the glpR gene or the nucleotide sequence encoding it is attenuated, in particular turned off, and the desired product isolated.

4. Process according to Claims 1, 2 or 3, **characterized in that** microorganisms are used in which the activity or concentration of the glpR gene product is reduced to 0 to 75% of the activity or concentration of the wild-type protein or in the parent strain.

5. Process according to Claims 1, 2 or 3, **characterized in that** expression of the polynucleotide which encodes the product of the glpR gene is attenuated, in particular turned off.

6. Process according to Claims 1, 2 or 3, **characterized in that** the regulatory and/or catalytic properties of the polypeptide (enzyme protein) which is encoded by the polynucleotide of the glpR gene are decreased.

7. Process according to Claims 1 to 6, **characterized in that**, for production of L-threonine, microorganisms of the family Enterobacteriaceae are fermented in which, in addition, at the same time, one or more of the genes selected from the group:
7.1 at least one gene of the thrABC operon encoding aspartate kinase, homoserine dehydrogenase, homoserine kinase and threonine synthase,
7.2 the pyc gene of Corynebacterium glutamicum encoding pyruvate carboxylase,
7.3 the pps gene encoding phosphoenolpyruvate synthase,
7.4 the ppc gene encoding phosphoenolpyruvate carboxylase,
7.5 the genes pntA and pntB encoding the subunits of pyridine transhydrogenase,
7.6 the gene rhtC encoding the protein imparting threonine resistance,
7.7 the thrE gene from Corynebacterium glutamicum encoding the threonine export carrier protein,
7.8 the gdhA gene encoding glutamate dehydrogenase,
7.9 the ptsH gene encoding phosphohistidineprotein-hexose phosphotransferase,
7.10 the ptsI gene encoding the enzyme I of the phosphotransferase system,
7.11 the crr gene encoding the glucose-specific IIA component,
7.12 the ptsG gene encoding the glucose-specific IIBC component,
7.13 the cysK gene encoding cysteine-synthase A,
7.14 the cysB gene encoding the regulator of the cys regulon,
7.15 the cysJ gene encoding the flavoprotein of NADPH-sulphite reductase,
7.16 the cysI gene encoding the haemoprotein of NADPH-sulphite reductase,
7.17 the cysH gene encoding adenylylsulphate-reductase,
7.18 the sucA gene encoding the decarboxylase subunit of 2-ketoglutarate dehydrogenase,
7.19 the sucB gene encoding the dihydrolipoyltranssuccinase E2 subunit of 2-ketoglutarate dehydrogenase,
7.20 the sucC gene encoding the β-subunit of succinyl-CoA synthetase,
7.21 the sucD gene encoding the α-subunit of succinyl-CoA synthetase, and
7.22 the gene product of the open reading frame (ORF) yibD of Escherichia coli
are amplified, in particular overexpressed.

8. Process according to Claims 1 to 6, **characterized in that**, for production of L-tryptophan, microorganisms of the family Enterobacteriaceae are fermented in which, in addition, at the same time, one or more of the genes selected from the group:
8.1 at least one gene of the tryptophan operon encoding anthranilate synthase, anthranilate phosphoribosyl transferase, phosphoribosylanthranilate isomerase, indole-3-glycerolphosphate synthase and tryptophan synthase,
8.2 the serA gene encoding 3-phosphoglycerate dehydrogenase,
8.3 the serB gene encoding phosphoserine phosphatase,
8.4 the serC gene encoding phosphoserine aminotransferase,
8.5 the aroF gene encoding L-tyrosine-sensitive DHAP synthase,
8.6 the aroG gene encoding L-phenylalaninesensitive DHAP synthase,
8.7 the aroH gene encoding L-tryptophan-sensitive DHAP synthase,
8.8 the pps gene encoding phosphoenolpyruvate synthase,
8.9 the pckA gene encoding phosphoenolpyruvate carboxykinase,
8.10 the tktA gene encoding transketolase A,
8.11 the tktB gene encoding transketolase B, and
8.12 the gene product of the open reading frame (ORF) yddG of Escherichia coli
are amplified, in particular overexpressed.

9. Process according to Claims 1 to 7, **characterized in that**, for production of L-threonine, microorganisms of the family Enterobacteriaceae are fermented in which, in addition, at the same time, one or more of the genes selected from the group:
9.1 the tdh gene encoding threonine dehydrogenase,
9.2 the mdh gene encoding malate dehydrogenase,
9.3 the gene product of the open reading frame (ORF) yjfA of Escherichia coli,
9.4 the gene product of the open reading frame (ORF) ytfP of Escherichia coli,
9.5 the pckA gene encoding phosphoenolpyruvate carboxykinase,
9.6 the poxB gene encoding pyruvate oxidase,
9.7 the dgsA gene encoding the DgsA regulator of the phosphotransferase system,
9.8 the fruR gene encoding the fructose repressor,
9.9 the rpoS gene encoding the Sigma³⁸ factor, and
9.10 the aspA gene encoding aspartate ammonium lyase,
are attenuated, in particular turned off, or the expression thereof is reduced.

10. Process according to Claims 1 to 6 or 8, **characterized in that**, for production of L-tryptophan, microorganisms of the family Enterobacteriaceae are fermented in which, in addition, at the same time, one or more of the genes selected from the group:
10.1 the tnaA gene encoding tryptophanase,
10.2 the trpR gene encoding the repressor of the trp operon,
10.3 the tyrA gene encoding chorismate mutase T and prephenate dehydrogenase,
10.4 the pheA gene encoding chorismate mutase P and prephenate dehydrogenase,
10.5 the mtr gene encoding the tryptophanspecific transport protein,
10.6 the tnaB gene encoding tryptophan permease,
10.7 the aroP gene encoding the transporter for aromatic amino acids,
10.8 the sdaA gene encoding L-serine deaminase,
10.9 the pgi gene encoding glucose-6-phosphate isomerase, and
10.10 the tyrB gene encoding tyrosine aminotransferase
are attenuated, in particular turned off, or the expression thereof is reduced.

11. Process according to Claims 1 to 6, **characterized in that** L-amino acids selected from the group L-asparagine, L-serine, L-glutamate, L-glycine, L-alanine, L-cysteine, L-valine, L-methionine, L-proline, L-isoleucine, L-leucine, L-tyrosine, L-phenylalanine, L-histidine, L-lysine, L-arginine and L-homoserine are produced.

12. Process according to Claims 1 to 6, **characterized in that** L-amino acids selected from the group L-isoleucine, L-valine, L-methionine, L-homoserine and L-lysine are produced.

## Revendications

1. Procédé pour la production d'acides aminés L, **caractérisé en ce qu'**on effectue les étapes suivantes :
a) culture par fermentation des micro-organismes appartenant à la famille des *Enterobacteriaceae,* produisant l'acide aminé L recherché, chez lesquels on affaiblit, en particulier inactive, le gène glpR ou la séquence nucléotidique codant pour celui-ci, dans un milieu contenant du glycérol et
b) accumulation de l'acide aminé L recherché dans le milieu ou dans les cellules des micro-organismes.

2. Procédé selon la revendication 1, **caractérisé en ce qu'**on isole l'acide aminé L recherché, les composants du bouillon de fermentation et/ou la biomasse éventuellement restant en leur totalité ou en parties (> 0 à 100 %) de ceux-ci dans le produit.

3. Procédé selon la revendication 1 ou 2 pour la production d'acides aminés L, en particulier de L-thréonine et L-tryptophane, ou d'additifs pour aliments pour animaux, contenant ces composés, par culture par fermentation de micro-organismes appartenant à la famille des *Enterobacteriaceae,* **caractérisé en ce que** chez ces derniers on affaiblit, en particulier inactive, le gène glpR ou la séquence nucléotidique codant pour celui-ci, et on isole le produit recherché.

4. Procédé selon les revendications 1, 2 ou 3, **caractérisé en ce qu'**on utilise des micro-organismes chez lesquels l'activité ou la concentration du produit du gène glpR est réduite à 0 à 75 % de l'activité ou de la concentration de la protéine de type sauvage ou chez la souche parentale.

5. Procédé selon les revendications 1, 2 ou 3, **caractérisé en ce qu'**on réduit, en particulier supprime, l'expression du polynucléotide qui code pour le produit du gène glpR.

6. Procédé selon les revendications 1, 2 ou 3, **caractérisé en ce qu'**on réduit les propriétés régulatrices et/ou catalytiques du polypeptide (protéine enzymatique) pour lequel code le polynucléotide du gène glpR.

7. Procédé selon les revendications 1 à 6, **caractérisé en ce que** pour la production de L-thréonine on cultive par fermentation des micro-organismes appartenant à la famille des *Enterobacteriaceae,* chez lesquels en outre on renforce, en particulier surexprime, simultanément un ou plusieurs des gènes choisis dans le groupe constitué par :
7.1 au moins un gène de l'opéron thrABC codant pour l'aspartate kinase, l'homosérine déshydrogénase, l'homosérine kinase et la thréonine synthase,
7.2 le gène pyc de *Corynebacterium glutamicum,* codant pour la pyruvate carboxylase,
7.3 le gène pps codant pour la phosphoénolpyruvate synthase,
7.4 le gène ppc codant pour la phosphoénolpyruvate carboxylase,
7.5 les gènes pntA et pntB codant pour les sous-unités de la pyridine transhydrogénase,
7.6 le gène rhtC codant pour la protéine conférant la résistance à la thréonine,
7.7 le gène thrE de *Corynebacterium glutamicum* codant pour la protéine export-carrier de thréonine,
7.8 le gène gdhA codant pour la glutamate déshydrogénase,
7.9 le gène ptsH codant pour la phosphohistidine-protéine-hexose phosphotransférase,
7.10 le gène ptsI codant pour l'enzyme I du système phosphotransférase,
7.11 le gène crr codant pour le composant IIA spécifique du glucose,
7.12 le gène ptsG codant pour le composant IIBC spécifique du glucose,
7.13 le gène cysK codant pour la cystéine synthase A,
7.14 le gène cysB codant pour le régulateur du régulon cys,
7.15 le gène cysJ codant pour la flavoprotéine de la NADPH-sulfite réductase,
7.16 le gène cysI codant pour l'hémoprotéine de la NADPH-sulfite réductase,
7.17 le gène cysH codant pour l'adénylylsulfate réductase,
7.18 le gène sucA codant pour la sous-unité décarboxylase de la 2-cétoglutarate déshydrogénase,
7.19 le gène sucB codant pour la sous-unité dihydrolipoyltrans-succinase E2 de la 2-cétoglutarate déshydrogénase,
7.20 le gène sucC codant pour la sous-unité β de la succinyl-CoA synthétase,
7.21 le gène sucD codant pour la sous-unité α de la succinyl-CoA synthétase,
7.22 le produit génique du cadre de lecture ouvert (ORF) yibD d'*Escherichia coli.*

8. Procédé selon les revendications 1 à 6, **caractérisé en ce que** pour la production de L-tryptophane on cultive par fermentation des micro-organismes appartenant à la famille des *Enterobacteriaceae,* chez lesquels en outre on renforce, en particulier surexprime, simultanément un ou plusieurs des gènes choisis dans le groupe constitué par :
8.1 au moins un gène de l'opéron tryptophane codant pour l'anthranilate synthase, l'anthranilate phosphoribosyltransférase, la phosphoribosylanthranilate isomérase, l'indole-3-glycérolphosphate synthase et la tryptophane synthase,
8.2 le gène serA codant pour la 3-phosphoglycérate déshydrogénase,
8.3 le gène serB codant pour la phosphosérine phosphatase,
8.4 le gène serC codant pour la phosphosérine aminotransférase,
8.5 le gène aroF codant pour la DHAP synthase sensible à la L-tyrosine,
8.6 le gène aroG codant pour la DHAP synthase sensible à la L-phénylalanine,
8.7 le gène aroH codant pour la DHAP synthase sensible au L-tryptophane,
8.8 le gène pps codant pour la phosphoénolpyruvate synthase,
8.9 le gène pckA codant pour la phosphoénolpyruvate carboxykinase,
8.10 le gène tktA codant pour la transcétolase A,
8.11 le gène tktB codant pour la transcétolase B, et
8.12 le produit génique du cadre de lecture ouvert (ORF) yddG d'*Escherichia coli.*

9. Procédé selon les revendications 1 à 7, **caractérisé en ce que** pour la production de L-thréonine on cultive par fermentation des micro-organismes appartenant à la famille des *Enterobacteriaceae,* chez lesquels en outre on affaiblit, en particulier inactive, simultanément un ou plusieurs des gènes choisis dans le groupe constitué par :
9.1 le gène tdh codant pour la thréonine déshydrogénase,
9.2 le gène mdh codant pour la malate déshydrogénase,
9.3 le produit génique du cadre de lecture ouvert (ORF) yjfA d'*Escherichia coli,*
9.4 le produit génique du cadre de lecture ouvert (ORF) ytfP d'*Escherichia coli,*
9.5 le gène pckA codant pour la phosphoénolpyruvate carboxykinase,
9.6 le gène poxB codant pour la pyruvate oxydase,
9.7 le gène dgsA codant pour le régulateur DgsA du système phosphotransférase,
9.8 le gène fruR codant pour le répresseur de fructose,
9.9 le gène rpoS codant pour le facteur Sigma³⁸, et
9.10 le gène aspA codant pour l'aspartate ammonium lyase,
ou diminue leur expression.

10. Procédé selon les revendications 1 à 6 ou 8, **caractérisé en ce que** pour la production de L-tryptophane on cultive par fermentation des micro-organismes appartenant à la famille des *Enterobacteriaceae,* chez lesquels en outre on affaiblit, en particulier inactive, simultanément un ou plusieurs des gènes choisis dans le groupe constitué par :
10.1 le gène tnaA codant pour la tryptophanase,
10.2 le gène trpR codant pour le répresseur de l'opéron trp,
10.3 le gène tyrA codant pour la chorismate mutase T et la préphénate déshydrogénase,
10.4 le gène pheA codant pour la chorismate mutase P et la préphénate déshydrogénase,
10.5 le gène mtr codant pour la protéine de transport spécifique du tryptophane,
10.6 le gène tnaB codant pour la tryptophane perméase,
10.7 le gène aroP codant pour le transporteur d'acides aminés aromatiques,
10.8 le gène sdaA codant pour la L-sérine désaminase,
10.9 le gène pgi codant pour la glucose-6-phosphate isomérase,
10.10 le gène tyrB codant pour la tyrosine aminotransférase
ou diminue leur expression.

11. Procédé selon les revendications 1 à 6, **caractérisé en ce qu'**on produit des acides aminés L, choisis dans le groupe constitué par la L-asparagine, la L-sérine, le L-glutamate, la L-glycine, la L-alanine, la L-cystéine, la L-valine, la L-méthionine, la L-proline, la L-isoleucine, la L-leucine, la L-tyrosine, la L-phénylalanine, la L-histidine, la L-lysine, la L-arginine et la L-homosérine.

12. Procédé selon les revendication 1 à 6, **caractérisé en ce qu'**on produit des acides aminés L, choisis dans le groupe constitué par la L-isoleucine, la L-valine, la L-méthionine, la L-homosérine et la L-lysine.


### SEQUENZPROTOKOLL

<110> Degussa AG
<120> Verfahren zur Herstellung von L-Aminosäuren unter Verwendung verbesserter Stämme der Familie Enterobacteriaceae
<130> 050067 BT
<160> 8
<170> PatentIn version 3.3
<210> 1
   <211> 759
   <212> DNA
   <213> Escherichia coli
<220>
   <221> CDS
   <222> (1)..(759)
   <223> glpR Kodierregion
<400> 1
   atg aaa caa aca caa cgt cac aac ggt att atc gaa ctg gtt aaa cag       48
   Met Lys Gln Thr Gln Arg His Asn Gly Ile Ile Glu Leu Val Lys Gln
   1               5                   10                  15
   cag ggt tat gtc agt acc gaa gag ctg gta gag cat ttc tcc gtc agc       96
   Gln Gly Tyr Val Ser Thr Glu Glu Leu Val Glu His Phe Ser Val Ser
               20                  25                  30
   ccg cag act att cgc cgc gac ctc aat gag ctg gcg gag caa aac ctg      144
   Pro Gln Thr Ile Arg Arg Asp Leu Asn Glu Leu Ala Glu Gln Asn Leu
           35                  40                  45
   atc ctg cgc cat cat ggc ggt gcg gcg ctg cct tcc agt tcg gtt aac      192
   Ile Leu Arg His His Gly Gly Ala Ala Leu Pro Ser Ser Ser Val Asn
       50                  55                  60
   acg ccg tgg cac gat cgc aag gcc acc cag acc gaa gaa aaa gag cgc      240
   Thr Pro Trp His Asp Arg Lys Ala Thr Gln Thr Glu Glu Lys Glu Arg
   65                  70                  75                  80
   atc gcc cgc aaa gtg gcg gag caa atc ccc aat ggc tcg acg ctg ttt      288
   Ile Ala Arg Lys Val Ala Glu Gln Ile Pro Asn Gly Ser Thr Leu Phe
                   85                  90                  95
   atc gat atc ggc acc acg ccg gaa gcg gta gcg cac gca ctg ctc aat      336
   Ile Asp Ile Gly Thr Thr Pro Glu Ala Val Ala His Ala Leu Leu Asn
               100                 105                 110
   cac agc aat ttg cgc att gtc acc aac aat ctc aac gtt gct aac acg      384
   His Ser Asn Leu Arg Ile Val Thr Asn Asn Leu Asn Val Ala Asn Thr
           115                 120                 125
   ttg atg gta aaa gaa gat ttt cgc atc att ctc gcc ggt ggc gaa tta      432
   Leu Met Val Lys Glu Asp Phe Arg Ile Ile Leu Ala Gly Gly Glu Leu
       130                 135                 140
   cgc agc cgc gat ggc ggg atc att ggc gaa gcg acg ctc gat ttt atc      480
   Arg Ser Arg Asp Gly Gly Ile Ile Gly Glu Ala Thr Leu Asp Phe Ile
   145                 150                 155                 160
   tcc cag ttc cgc ctt gat ttc ggc att ctg ggg ata agc ggc atc gat      528
   Ser Gln Phe Arg Leu Asp Phe Gly Ile Leu Gly Ile Ser Gly Ile Asp
                   165                 170                 175
   agc gac ggc tcg ctg ctg gag ttc gat tac cac gaa gtt cgc acc aaa      576
   Ser Asp Gly Ser Leu Leu Glu Phe Asp Tyr His Glu Val Arg Thr Lys
               180                 185                 190
   cgc gcc att att gag aac tcg cgc cac gtt atg ctg gtt gtc gat cac      624
   Arg Ala Ile Ile Glu Asn Ser Arg His Val Met Leu Val Val Asp His
           195                 200                 205
   tcg aaa ttt ggc cgt aac gcg atg gtc aat atg ggc agc atc agc atg      672
   Ser Lys Phe Gly Arg Asn Ala Met Val Asn Met Gly Ser Ile Ser Met
       210                 215                 220
   gta gat gcc gtc tac acc gac gcc ccg ccg cca gta agc gtg atg cag      720
   Val Asp Ala Val Tyr Thr Asp Ala Pro Pro Pro Val Ser Val Met Gln
   225                 230                 235                 240
   gtg ctg acg gac cac cat att caa ctg gag ctg tgc tga                  759
   Val Leu Thr Asp His His Ile Gln Leu Glu Leu Cys
                   245                 250
<210> 2
   <211> 252
   <212> PRT
   <213> Escherichia coli
<400> 2
   Met Lys Gln Thr Gln Arg His Asn Gly Ile Ile Glu Leu Val Lys Gln
   1               5                   10                  15
   Gln Gly Tyr Val Ser Thr Glu Glu Leu Val Glu His Phe Ser Val Ser
               20                  25                  30
   Pro Gln Thr Ile Arg Arg Asp Leu Asn Glu Leu Ala Glu Gln Asn Leu
           35                  40                  45
   Ile Leu Arg His His Gly Gly Ala Ala Leu Pro Ser Ser Ser Val Asn
       50                  55                  60
   Thr Pro Trp His Asp Arg Lys Ala Thr Gln Thr Glu Glu Lys Glu Arg
   65                  70                  75                  80
   Ile Ala Arg Lys Val Ala Glu Gln Ile Pro Asn Gly Ser Thr Leu Phe
                   85                  90                  95
   Ile Asp Ile Gly Thr Thr Pro Glu Ala Val Ala His Ala Leu Leu Asn
               100                 105                 110
   His Ser Asn Leu Arg Ile Val Thr Asn Asn Leu Asn Val Ala Asn Thr
           115                 120                 125
   Leu Met Val Lys Glu Asp Phe Arg Ile Ile Leu Ala Gly Gly Glu Leu
       130                 135                 140
   Arg Ser Arg Asp Gly Gly Ile Ile Gly Glu Ala Thr Leu Asp Phe Ile
   145                 150                 155                 160
   Ser Gln Phe Arg Leu Asp Phe Gly Ile Leu Gly Ile Ser Gly Ile Asp
                   165                 170                 175
   Ser Asp Gly Ser Leu Leu Glu Phe Asp Tyr His Glu Val Arg Thr Lys
               180                 185                 190
   Arg Ala Ile Ile Glu Asn Ser Arg His Val Met Leu Val Val Asp His
           195                 200                 205
   Ser Lys Phe Gly Arg Asn Ala Met Val Asn Met Gly Ser Ile Ser Met
       210                 215                 220
   Val Asp Ala Val Tyr Thr Asp Ala Pro Pro Pro Val Ser Val Met Gln
   225                 230                 235                 240
   Val Leu Thr Asp His His Ile Gln Leu Glu Leu Cys
                   245                 250
<210> 3
   <211> 759
   <212> DNA
   <213> Salmonella typhimurium
<220>
   <221> CDS
   <222> (1)..(759)
   <223> glpR Kodierregion
<400> 3
   atg aaa caa aca caa cgg cat gac gcg atc att gaa ctg gta aaa aaa       48
   Met Lys Gln Thr Gln Arg His Asp Ala Ile Ile Glu Leu Val Lys Lys
   1               5                   10                  15
   cag ggg tac gtc agt acg gaa gag ctg gtg gag cat ttt tct gtc agc       96
   Gln Gly Tyr Val Ser Thr Glu Glu Leu Val Glu His Phe Ser Val Ser
               20                  25                  30
   ccg caa acc att cgt cgg gat ctt aac gat ctg gcg gaa cag aat atg      144
   Pro Gln Thr Ile Arg Arg Asp Leu Asn Asp Leu Ala Glu Gln Asn Met
           35                  40                  45
   att ttg cgc cac cac ggc ggc gcg gcg ttg ccc tcc agc tcg gtg aac      192
   Ile Leu Arg His His Gly Gly Ala Ala Leu Pro Ser Ser Ser Val Asn
       50                  55                  60
   acg ccg tgg cac gat cgt aag gcg acg caa acg gaa gaa aaa gag cgc      240
   Thr Pro Trp His Asp Arg Lys Ala Thr Gln Thr Glu Glu Lys Glu Arg
   65                  70                  75                  80
   atc gct cgc aaa gtc gcg gcc cag atc ccc aat ggt tca acg ctg ttt      288
   Ile Ala Arg Lys Val Ala Ala Gln Ile Pro Asn Gly Ser Thr Leu Phe
                   85                  90                  95
   att gat atc ggc acg acg ccg gaa gcc gta gcg cac gcc ctg tta ggg      336
   Ile Asp Ile Gly Thr Thr Pro Glu Ala Val Ala His Ala Leu Leu Gly
           100                 105                 110
   cac agt aat ttg cgg atc gtg acc aac aat ttg aac gta gcg aac acg     384
   His Ser Asn Leu Arg Ile Val Thr Asn Asn Leu Asn Val Ala Asn Thr
       115                 120                 125
   cta atg gcg aaa gag gat ttt cgt att atc ctc gcc ggc ggt gaa ctc     432
   Leu Met Ala Lys Glu Asp Phe Arg Ile Ile Leu Ala Gly Gly Glu Leu
       130                 135                 140
   cgt agc cgc gac ggc ggc att att ggc gaa gcg acg cag gac ttt atc     480
   Arg Ser Arg Asp Gly Gly Ile Ile Gly Glu Ala Thr Gln Asp Phe Ile
   145                 150                 155                 160
   gcc cag ttc cgt ctc gat ttc ggc att ctc ggt att agc ggt att gat     528
   Ala Gln Phe Arg Leu Asp Phe Gly Ile Leu Gly Ile Ser Gly Ile Asp
                   165                 170                 175
   agc gac ggc tcg ttg ctg gaa ttt gac tac cat gag gta cgc acc aag     576
   Ser Asp Gly Ser Leu Leu Glu Phe Asp Tyr His Glu Val Arg Thr Lys
               180                 185                 190
   cgc gcg att atc gaa aat tcg cgc cac gtg atg ctg gtg gtg gat cac     624
   Arg Ala Ile Ile Glu Asn Ser Arg His Val Met Leu Val Val Asp His
           195                 200                 205
   tct aaa ttt ggc cgt aac gcg atg gta aac atg ggc agc att agt atg     672
   Ser Lys Phe Gly Arg Asn Ala Met Val Asn Met Gly Ser Ile Ser Met
       210                 215                 220
   gtc gat gcg gtc tac acc gat acc ctg ccg ccg ccg ggc gtg atg cag     720
   Val Asp Ala Val Tyr Thr Asp Thr Leu Pro Pro Pro Gly Val Met Gln
   225                 230                 235                 240
   gtg ttg acg gag aac cat att caa ctg gag ctg tgt taa                 759
   Val Leu Thr Glu Asn His Ile Gln Leu Glu Leu Cys
                   245                 250
<210> 4
   <211> 252
   <212> PRT
   <213> Salmonella typhimurium
<400> 4
   Met Lys Gln Thr Gln Arg His Asp Ala Ile Ile Glu Leu Val Lys Lys
   1               5                   10                  15
   Gln Gly Tyr Val Ser Thr Glu Glu Leu Val Glu His Phe Ser Val Ser
               20                  25                  30
   Pro Gln Thr Ile Arg Arg Asp Leu Asn Asp Leu Ala Glu Gln Asn Met
           35                  40                  45
   Ile Leu Arg His His Gly Gly Ala Ala Leu Pro Ser Ser Ser Val Asn
       50                  55                  60
   Thr Pro Trp His Asp Arg Lys Ala Thr Gln Thr Glu Glu Lys Glu Arg
   65                  70                  75                  80
   Ile Ala Arg Lys Val Ala Ala Gln Ile Pro Asn Gly Ser Thr Leu Phe
                   85                  90                  95
   Ile Asp Ile Gly Thr Thr Pro Glu Ala Val Ala His Ala Leu Leu Gly
               100                 105                 110
   His Ser Asn Leu Arg Ile Val Thr Asn Asn Leu Asn Val Ala Asn Thr
           115                 120                 125
   Leu Met Ala Lys Glu Asp Phe Arg Ile Ile Leu Ala Gly Gly Glu Leu
       130                 135                 140
   Arg Ser Arg Asp Gly Gly Ile Ile Gly Glu Ala Thr Gln Asp Phe Ile
   145                 150                 155                 160
   Ala Gln Phe Arg Leu Asp Phe Gly Ile Leu Gly Ile Ser Gly Ile Asp
                   165                 170                 175
   Ser Asp Gly Ser Leu Leu Glu Phe Asp Tyr His Glu Val Arg Thr Lys
               180                 185                 190
   Arg Ala Ile Ile Glu Asn Ser Arg His Val Met Leu Val Val Asp His
           195                 200                 205
   Ser Lys Phe Gly Arg Asn Ala Met Val Asn Met Gly Ser Ile Ser Met
       210                 215                 220
   Val Asp Ala Val Tyr Thr Asp Thr Leu Pro Pro Pro Gly Val Met Gln
   225                 230                 235                 240
   Val Leu Thr Glu Asn His Ile Gln Leu Glu Leu Cys
                   245                 250
<210> 5
   <211> 759
   <212> DNA
   <213> Shigella flexneri
<220>
   <221> CDS
   <222> (1) .. (759)
   <223> glpR Kodierregion
<400> 5
   atg aaa caa aca caa cgt cac aac ggt att atc gaa ctg gtt aaa cag       48
   Met Lys Gln Thr Gln Arg His Asn Gly Ile Ile Glu Leu Val Lys Gln
   1               5                   10                  15
   cag ggt tat gtc agt acc gag gaa ctg gtg gag cac ttc tcc gtc agc       96
   Gln Gly Tyr Val Ser Thr Glu Glu Leu Val Glu His Phe Ser Val Ser
               20                  25                  30
   ccg cag act att cgc cgc gac ctc aac gag ctg gca gag caa aac ctg      144
   Pro Gln Thr Ile Arg Arg Asp Leu Asn Glu Leu Ala Glu Gln Asn Leu
           35                  40                  45
   atc ctg cgc cat cat ggc ggc gcg gcg ctg cct tcc agt tcg gtt aac      192
   Ile Leu Arg His His Gly Gly Ala Ala Leu Pro Ser Ser Ser Val Asn
       50                  55                  60
   acg ccg tgg cac gat cgt aaa gcc acc cag acc gaa gaa aaa gag cgc      240
   Thr Pro Trp His Asp Arg Lys Ala Thr Gln Thr Glu Glu Lys Glu Arg
   65                  70                  75                  80
   atc gcc cgc aaa gtg gcg gag caa atc ccc aat ggc tcg acg ctg ttt      288
   Ile Ala Arg Lys Val Ala Glu Gln Ile Pro Asn Gly Ser Thr Leu Phe
                   85                  90                  95
   atc gat atc ggc acc acg ccg gaa gcg gtg gcg cac gcg ctg ctc aat      336
   Ile Asp Ile Gly Thr Thr Pro Glu Ala Val Ala His Ala Leu Leu Asn
               100                 105                 110
   cac agc aat ttg cgc att gtc acc aac aat ctc aac gtt gct aac acg      384
   His Ser Asn Leu Arg Ile Val Thr Asn Asn Leu Asn Val Ala Asn Thr
           115                 120                 125
   ttg atg gta aaa gaa gat ttc cgc att att ctc gcc ggt ggc gaa tta      432
   Leu Met Val Lys Glu Asp Phe Arg Ile Ile Leu Ala Gly Gly Glu Leu
       130                 135                 140
   cgt agc cgc gat ggc ggg atc att ggc gaa gcg acg ctc gat ttt atc      480
   Arg Ser Arg Asp Gly Gly Ile Ile Gly Glu Ala Thr Leu Asp Phe Ile
   145                 150                 155                 160
   tcc cag ttc cgc ctt gat ttc ggc att ctg ggg atc agc ggc atc gac      528
   Ser Gln Phe Arg Leu Asp Phe Gly Ile Leu Gly Ile Ser Gly Ile Asp
                   165                 170                 175
   agc gac ggc tcg ctg ctg gag ttc gat tac cac gaa gtt cgt acc aag      576
   Ser Asp Gly Ser Leu Leu Glu Phe Asp Tyr His Glu Val Arg Thr Lys
               180                 185                 190
   cgc gcc att att gag aac tcg cgc cac gtt atg ctg gtg gtt gac cat      624
   Arg Ala Ile Ile Glu Asn Ser Arg His Val Met Leu Val Val Asp His
           195                 200                 205
   tca aaa ttt ggc cgt aat gcg atg gtc aat atg ggg agc atc agc atg      672
   Ser Lys Phe Gly Arg Asn Ala Met Val Asn Met Gly Ser Ile Ser Met
       210                 215                 220
   gtt gat gcc gtc tac acc gac gcc ccg ccg cca gta agc gtg atg cag      720
   Val Asp Ala Val Tyr Thr Asp Ala Pro Pro Pro Val Ser Val Met Gln
   225                 230                 235                 240
   gtg ctg acg gac cac cat att caa ctg gag ctg tgc tga                  759
   Val Leu Thr Asp His His Ile Gln Leu Glu Leu Cys
                   245                 250
<210> 6
   <211> 252
   <212> PRT
   <213> Shigella flexneri
<400> 6
   Met Lys Gln Thr Gln Arg His Asn Gly Ile Ile Glu Leu Val Lys Gln
   1               5                   10                  15
   Gln Gly Tyr Val Ser Thr Glu Glu Leu Val Glu His Phe Ser Val Ser
               20                  25                  30
   Pro Gln Thr Ile Arg Arg Asp Leu Asn Glu Leu Ala Glu Gln Asn Leu
           35                  40                  45
   Ile Leu Arg His His Gly Gly Ala Ala Leu Pro Ser Ser Ser Val Asn
       50                  55                  60
   Thr Pro Trp His Asp Arg Lys Ala Thr Gln Thr Glu Glu Lys Glu Arg
   65                  70                  75                  80
   Ile Ala Arg Lys Val Ala Glu Gln Ile Pro Asn Gly Ser Thr Leu Phe
                   85                  90                  95
   Ile Asp Ile Gly Thr Thr Pro Glu Ala Val Ala His Ala Leu Leu Asn
               100                 105                 110
   His Ser Asn Leu Arg Ile Val Thr Asn Asn Leu Asn Val Ala Asn Thr
           115                 120                 125
   Leu Met Val Lys Glu Asp Phe Arg Ile Ile Leu Ala Gly Gly Glu Leu
       130                 135                 140
   Arg Ser Arg Asp Gly Gly Ile Ile Gly Glu Ala Thr Leu Asp Phe Ile
   145                 150                 155                 160
   Ser Gln Phe Arg Leu Asp Phe Gly Ile Leu Gly Ile Ser Gly Ile Asp
                   165                 170                 175
   Ser Asp Gly Ser Leu Leu Glu Phe Asp Tyr His Glu Val Arg Thr Lys
               180                 185                 190
   Arg Ala Ile Ile Glu Asn Ser Arg His Val Met Leu Val Val Asp His
           195                 200                 205
   Ser Lys Phe Gly Arg Asn Ala Met Val Asn Met Gly Ser Ile Ser Met
       210                 215                 220
   Val Asp Ala Val Tyr Thr Asp Ala Pro Pro Pro Val Ser Val Met Gln
   225                 230                 235                 240
   Val Leu Thr Asp His His Ile Gln Leu Glu Leu Cys
                   245                 250
<210> 7
   <211> 759
   <212> DNA
   <213> Erwinia carotovora
<220>
   <221> CDS
   <222> (1)..(759)
   <223> glpR Kodierregion
<400> 7
   gtg aag cag aca caa cgg cat gac gcc att att gaa ctg gtg cgt cgg        48
   Met Lys Gln Thr Gln Arg His Asp Ala Ile Ile Glu Leu Val Arg Arg
   1               5                   10                  15
   cag ggg tat gtc agt act gaa gaa ctg gtg gat cat ttt gcg gtg agc        96
   Gln Gly Tyr Val Ser Thr Glu Glu Leu Val Asp His Phe Ala Val Ser
               20                  25                  30
   ccg cag acc atc cgt cgc gat ctg aac gat ctg gct gaa cag aat aaa       144
   Pro Gln Thr Ile Arg Arg Asp Leu Asn Asp Leu Ala Glu Gln Asn Lys
           35                  40                  45
   atc cat cgt cac cac ggt ggt gcg gct ttg ccg tcc agt tca gtt aac       192
   Ile His Arg His His Gly Gly Ala Ala Leu Pro Ser Ser Ser Val Asn
       50                  55                  60
   acg gct tat cat gac cgt aaa atg atg tgg tcg gat gaa aaa gcg cgc       240
   Thr Ala Tyr His Asp Arg Lys Met Met Trp Ser Asp Glu Lys Ala Arg
   65                  70                  75                  80
   att gcc cgt cgg gtg gcg agc cag att cca gac ggg gct acc ttg ttt       288
   Ile Ala Arg Arg Val Ala Ser Gln Ile Pro Asp Gly Ala Thr Leu Phe
                   85                  90                  95
   atc gac atc ggc acc acg ccc gaa gcg gtg gcc tat gcg ctg atg cag       336
   Ile Asp Ile Gly Thr Thr Pro Glu Ala Val Ala Tyr Ala Leu Met Gln
               100                 105                 110
   cat aag gat ctg cgt gtg gtc acc aat aac ctg aat gtg gca aca ctg       384
   His Lys Asp Leu Arg Val Val Thr Asn Asn Leu Asn Val Ala Thr Leu
           115                 120                 125
   ctg act gca aaa gag gat ttt cgc ctg att ttg gct ggt ggc gaa gtg       432
   Leu Thr Ala Lys Glu Asp Phe Arg Leu Ile Leu Ala Gly Gly Glu Val
       130                 135                 140
   cgg acc cgc gat ggc ggt att atg ggg gag gcg acg ctc gat ttt atc       480
   Arg Thr Arg Asp Gly Gly Ile Met Gly Glu Ala Thr Leu Asp Phe Ile
   145                 150                 155                 160
   tct cag ttc cgt ctg gat ttc ggc att ttg ggc atc agc ggt att gac       528
   Ser Gln Phe Arg Leu Asp Phe Gly Ile Leu Gly Ile Ser Gly Ile Asp
                   165                 170                 175
   atg gat ggg tca tta ctg gag ttt gat tac cat gaa gtg cgt aca aaa       576
   Met Asp Gly Ser Leu Leu Glu Phe Asp Tyr His Glu Val Arg Thr Lys
               180                 185                 190
   cgg gcg att att gaa aat tct cgc tgc gtc atg ctg gtg aca gat cat       624
   Arg Ala Ile Ile Glu Asn Ser Arg Cys Val Met Leu Val Thr Asp His
           195                 200                 205
   tcc aag ttc ggc cgc aat gcg atg gtg aat ttg ggt aac atg gat ttg       672
   Ser Lys Phe Gly Arg Asn Ala Met Val Asn Leu Gly Asn Met Asp Leu
       210                 215                 220
   atc gac tat ctt ttt acc gac cag tca cca cca ccc agc gta ctg aaa       720
   Ile Asp Tyr Leu Phe Thr Asp Gln Ser Pro Pro Pro Ser Val Leu Lys
   225                 230                 235                 240
   atc att gaa caa cat aag gta cag ttg gag ttg tgt taa                 759
   Ile Ile Glu Gln His Lys Val Gln Leu Glu Leu Cys
                   245                 250
<210> 8
   <211> 252
   <212> PRT
   <213> Erwinia carotovora
<400> 8
   Val Lys Gln Thr Gln Arg His Asp Ala Ile Ile Glu Leu Val Arg Arg
   1               5                   10                  15
   Gln Gly Tyr Val Ser Thr Glu Glu Leu Val Asp His Phe Ala Val Ser
               20                  25                  30
   Pro Gln Thr Ile Arg Arg Asp Leu Asn Asp Leu Ala Glu Gln Asn Lys
           35                  40                  45
   Ile His Arg His His Gly Gly Ala Ala Leu Pro Ser Ser Ser Val Asn
       50                  55                  60
   Thr Ala Tyr His Asp Arg Lys Met Met Trp Ser Asp Glu Lys Ala Arg
   65                  70                  75                  80
   Ile Ala Arg Arg Val Ala Ser Gln Ile Pro Asp Gly Ala Thr Leu Phe
                   85                  90                  95
   Ile Asp Ile Gly Thr Thr Pro Glu Ala Val Ala Tyr Ala Leu Met Gln
               100                 105                 110
   His Lys Asp Leu Arg Val Val Thr Asn Asn Leu Asn Val Ala Thr Leu
           115                 120                 125
   Leu Thr Ala Lys Glu Asp Phe Arg Leu Ile Leu Ala Gly Gly Glu Val
       130                 135                 140
   Arg Thr Arg Asp Gly Gly Ile Met Gly Glu Ala Thr Leu Asp Phe Ile
   145                 150                 155                 160
   Ser Gln Phe Arg Leu Asp Phe Gly Ile Leu Gly Ile Ser Gly Ile Asp
                   165                 170                 175
   Met Asp Gly Ser Leu Leu Glu Phe Asp Tyr His Glu Val Arg Thr Lys
               180                 185                 190
   Arg Ala Ile Ile Glu Asn Ser Arg Cys Val Met Leu Val Thr Asp His
           195                 200                 205
   Ser Lys Phe Gly Arg Asn Ala Met Val Asn Leu Gly Asn Met Asp Leu
           210                 215                 220
   Ile Asp Tyr Leu Phe Thr Asp Gln Ser Pro Pro Pro Ser Val Leu Lys
   225                 230                 235                 240
   Ile Ile Glu Gln His Lys Val Gln Leu Glu Leu Cys
                   245                 250